# EUROPEAN PATENT SPECIFICATION

(11) **EP 3 076 987 B1**
(45) Date of publication and mention of the grant of the patent: **12.07.2023**
(21) Application number: 14867603.4
(22) Date of filing: 04.12.2014
(51) Int. Cl.: A61K 38/10, A61P 25/08

(54) **METHOD OF TREATING OR PREVENTING EPILEPSY OR SEIZURES USING ONE OR MORE NEURAL REGENERATION PEPTIDES**
VERFAHREN ZUR BEHANDLUNG ODER PRÄVENTION VON EPILEPSIE ODER ANFÄLLEN MITHILFE EINER ODER MEHRERER PEPTIDE ZUR NEURALEN REGENERATION
MÉTHODE DE TRAITEMENT OU DE PRÉVENTION DE L'ÉPILEPSIE OU DES CRISES D'ÉPILEPSIE FAISANT APPEL À UN OU PLUSIEURS PEPTIDES STIMULANT LA RÉGÉNÉRATION NEURONALE

(30) Priority: 04.12.2013 NZ 61861413
(43) Date of publication of application: 12.10.2016
(73) Proprietor: Curonz Holdings Company Limited, Auckland (NZ)
(72) Inventor: SIEG, Frank, Wellsford, 0975 (NZ)
(74) Representative: Mewburn Ellis LLP
(86) International application number: PCT/NZ2014/050016
(87) International publication number: WO 2015/084188

(56) References cited:
- WO-A1-2009/051844
- WO-A2-2007/011595
- US-A1- 2005 131 212
- US-A1- 2010 130 410
- US-A1- 2012 100 116
- US-A1- 2013 231 289
- FROESTL WOLFGANG ET AL: "Cognitive Enhancers (Nootropics). Part 3: Drugs Interacting with Targets other than Receptors or Enzymes. Disease-modifying Drugs", JOURNAL OF ALZHEIMER'S DISEASE, IOS PRESS, NL, vol. 34, 1 January 2013 (2013-01-01), pages 1-114, XP009186604, ISSN: 1387-2877, DOI: 10.3233/JAD-121729

## Description

### FIELD OF INVENTION

The invention relates to a peptide comprising the sequence:
GlyArgArgAlaAlaProGlyArgAibGlyGly (SEQ ID NO:1) for use in a method of preventing (i) epilepsy; or (ii)a seizure; which method comprises administering an effective amount of the peptide to a subject in need thereof, as defined in the claims.

### BACKGROUND ART

Neural Regeneration Peptides (NRPs) are small synthetic peptides that promote proliferation, migration, differentiation and survival of neural precursor and committed neural stem cells under conditions of central nervous system disease or injury (Gorba et al., 2006; Singh et al., 2010). The discovery of NRPs dates back to experimentation with a developed in vitro model of traumatic brain injury (Sieg et al., 1999). Administration of biochemically fractionated cell culture supernatant derived from hippocampal organotypic tissue cultures (OTCs) led to a neuronal bridge formation in thalamocortical OTC cocultures within 3-4 days (Landgraf et al., 2005). The first completely identified gene that encodes for bioactive peptides displaying femtomolar potency for neuronal survival-promoting activity was experimentally shown to be encoded on chromosome 12 in mice and is coding for a 135 amino acid long protein (Gorba et al., 2006). NRP2945 derives from the human NRP gene located at position 7q31.35 also known as calcium dependent activator of protein secretion 2 (CAPS2; Sadakata et al., 2004) and represents amino acid position 40-50 within the CAPS2 sequence (Speidel et al., 2003).

NRPs have both neurodegenerative and anti-inflammatory effects (Landgraf et al. 2005; Gorba et al., 2006 and Sieg & Antonic, 2007). Several studies using human primary brain cells, human embryonic neural stem cells and animal cells subsequently confirmed that NRPs at sub-nanomolar concentrations have both neuroprotective and anti-inflammatory efficacy suitable for the treatment of neuroinflammatory diseases (Gorba et al., 2006).

Over the past two decades, an increasing amount of evidence indicates that activation of inflammatory processes occurs during epileptogenesis which contribute in concert to the adverse outcomes. Increased levels of inflammatory molecules and upregulation of their receptors in glial and neuronal cells as well as microvascular endothelial cells have been demonstrated in human brain tissues of medically refractory epileptic patients; suggesting that proinflammatory pathways are activated in seizure foci (Aronica et al., 2012, Choi et al., 2009 and Vezzani et al., 2011a).

There is a need for neuroprotective and anti-inflammatory drugs that may decrease the expression of inflammatory molecules and cell loss in the brain and possibly slow the rate of epileptogenesis.

It is an object to provide a method for treating or preventing epilepsy or a seizure or epilepsy or seizure damage in a subject using a neural regeneration peptide or to at least-te provide a useful choice.

US 2005/131212 A1 discloses a family of peptides termed NRP compounds or NRPs that can promote neuronal migration, neurite outgrowth, neuronal proliferation, neural differentiation and/or neuronal survival and provides compositions and methods for the use of NRPs in the treatment of brain injury and neurodegenerative disease.

WO 2009/051844 A1 discloses synthetic compounds (NRP analogues) of NRPs and their use in treating a variety of conditions involving degeneration of neural cells, including treating disorders of the nervous system such as peripheral neuropathy, multiple sclerosis, diabetic peripheral neuropathy, neurotoxin-induced neurodegeneration, and amyotrophic lateral sclerosis.

FroestI et al. ("Cognitive Enhancers (Nootropics). Part 3: Drugs Interacting with Targets other than Receptors or Enzymes. Disease-modifying drugs", J Alzheimers Dis. 2013;34(1):1-114.) is a review article that assigns cognitive enhancers (nootropics) to various categories. Peptides NNZ-4921 and NNZ-4945 are disclosed as neuronal regeneration peptides for the potential treatment of multiple sclerosis and motor neuron disease, respectively.

### STATEMENT OF INVENTION

In a first aspect, the invention provides a peptide comprising the sequence: GlyArgArgAlaAlaProGlyArgAibGlyGly (SEQ ID NO:1) for use in a method of preventing
(i) epilepsy; or
(ii) a seizure;
which method comprises administering an effective amount of the peptide to a subject in need thereof.

In one embodiment, the peptide consists of the 11 amino acid residue sequence:
GlyArgArgAlaAlaProGlyArgAibGlyGly and functionally acceptable derivatives thereof. One such acceptable derivative includes the sequence wherein the C-terminus of the peptide is amidated to give: GlyArgArgAlaAlaProGlyArgAibGlyGly-NH₂(SEQ ID NO:2).

In one embodiment, the administration step to the subject is by way of injection, including but not limited to intraperitoneal, intravenous, intramuscular, intraarterial, subcutaneous, intradermal, intracerebroventricular, intranasal and intraspinal injection. In another embodiment, the administration step to the subject is by way of oral administration.

In another embodiment, the effective amount of the NRP is at least 5µg/kg administered to the subject. In another embodiment, the effective amount of the NRP is between about 10-50µg/kg of the NRP administered to the subject.

In one embodiment, the peptid for use defined above includes the step of administering an effective amount of a peptide comprising the sequence:
GlyArgArgAlaAlaProGlyArgAibGlyGly (SEQ ID NO:1)or
GlyArgArgAlaAlaProGlyArgAibGlyGly-NH₂ (SEQ ID NO:2)
to a subject in need thereof on an at least once a day basis.

In one embediment the peptid for use includes the step of administering an effective amount of a peptide comprising the sequence:
GlyArgArgAlaAlaProGlyArgAibGlyGly (SEQ ID NO:1) to a subject in need thereof on an at least once a day basis.

In one embodiment, the peptid for use defined above includes the step of administering the peptide on an at least every-other-day basis.

In one embodiment, the subject is selected from the group consisting of: humans and companion animals.

In one embodiment, the administration step to the subject is by way of injection, including but not limited to intraperitoneal, intravenous, intramuscular, intraarterial, subcutaneous, intradermal, intracerebroventricular, intranasal and intraspinal injection. In another embodiment, the administration step to the subject is by way of oral administration.

In another embodiment, the effective amount of the NRP is at least 5µg/kg administered to the subject. In another embodiment, the effective amount of the NRP is between about 10-50µg/kg of the NRP administered to the subject.

It is to be understood from the following description that the effective amount of the peptide of SEQ ID NO:1 or SEQ ID NO: 2 is indicated to be in the range of 5-20µg/kg dose amounts when administered in rodent models and in the range of 5-50 µg/kg dose amounts when administered to larger subjects such as dogs or humans.

In the description and claims of this specification the following acronyms, terms and phrases have the meaning provided:
"Effective amount" means an amount effective to treat or prevent epilepsy or seizure damage arising in a subject as a result of epilepsy or seizure activity in the subject.

"Functionally acceptable derivatives" means derivatives of the peptide defined in SEQ ID NO:1 obtained by amidation, acylation, alkylation, carboxylation, glycosylation, phosphorylation, prenylation, salification, sulfation, or a combination thereof, that are suitable for inclusion in a composition for administration to a subject either before or after epilepsy or seizure activity in the subject.

"Epilepsy damage" means neuronal damage in the brain of a subject caused by the process of epileptogenesis.

"Seizure damage" means neuronal damage in the brain of a subject caused by a seizure or convulsion resulting from the process of epileptogenesis.

The invention will now be described with reference to embodiments or examples and the figures of the accompanying drawings pages.

### BRIEF DESCRIPTION OF DRAWINGS

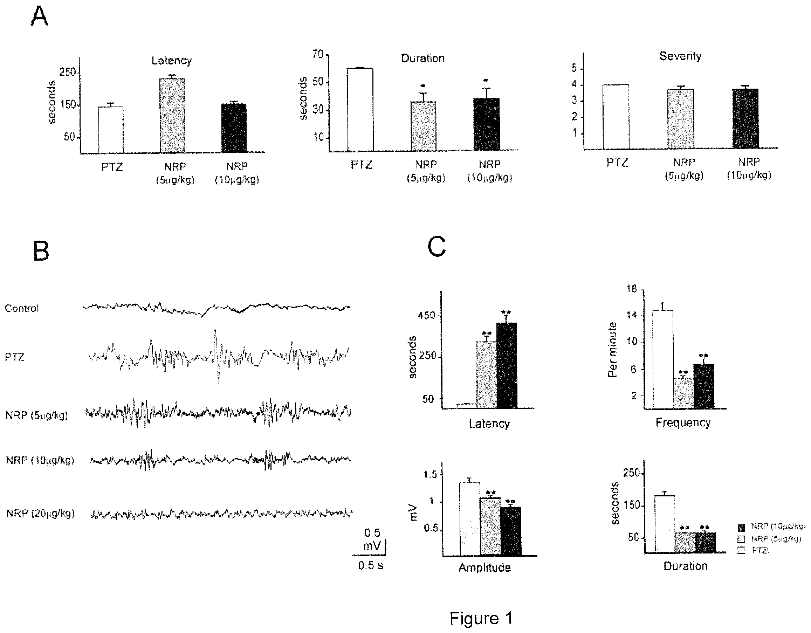

Figure 1A. Shows plots of the effects upon duration, latency and severity (assessed by behavioural observation) of seizure attacks in rats where SEQ ID NO: 1, depicted as NRP2945 in the accompanying Figures and Description, at concentrations of 5µg/kg, 10µg/kg, when administered by way of prophylactic intra peritoneal injection in the rats that were then exposed to PTZ to induce seizures compared to an injury control group.
Figure 1B. Shows representative examples of epileptiform burst discharges recorded by electrocorticography (ECoG) in the neocortices of control rats (sham operated) and rats intoxicated with PTZ as well as animals treated with different concentrations of NRP2945 (5µg/kg, 10pg/kg and 20 µg/kg), was administered by way of prophylactic intra peritoneal injection in the rats that were then exposed to PTZ to induce seizures compared to an injury control group.
Figure 1C. Shows plots of the effect of NRP2945 at concentrations of 5 and 10 µg/Kg on the latency, frequency, amplitude and duration of epileptiform burst discharges induced by PTZ injection assessed by ECoG recordings. The amplitude of spikes was significantly smaller and the duration of spikes was significantly shorter in rats treated with 5 and 10 µg/kg compared to control animals. The frequency of burst discharges was significantly lower than control rats.* and ** indicate p<0.05 and p<0.001, respectively.

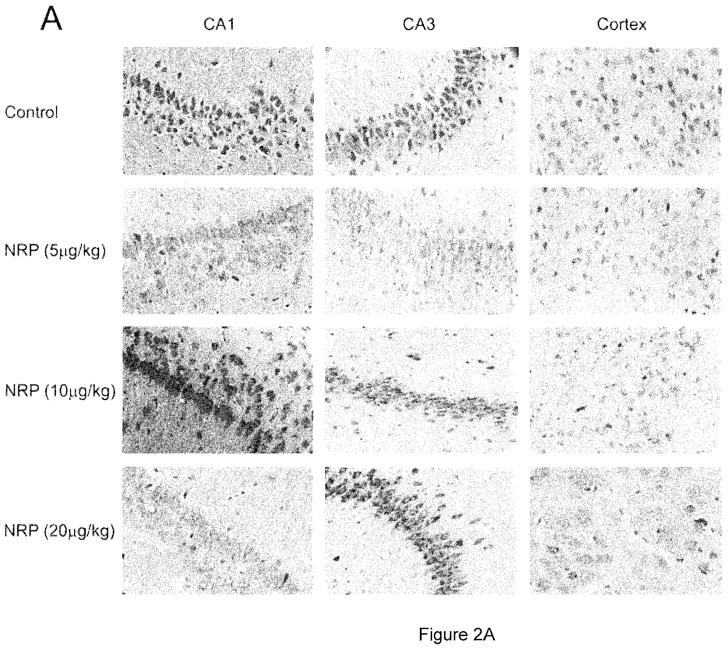

Figure 2A. Shows various photos illustrating the inhibitory effect of NRP2945 administration on production of dark neurons and the light-microscopic appearance of toluidine blue stained dark neurons in 8-µm sections of the hippocampal CA1 and CA3 areas as well as the neocortex in rats.

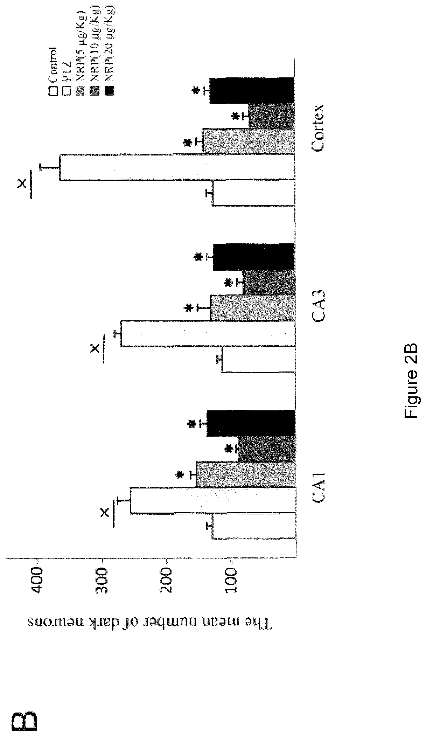

Figure 2B. Shows a plot of the inhibitory effect of NRP2945 administration on production of dark neurons against the effects of NRP2945 at concentrations of 5, 10, and 20 µg/kg. NRP 2945 inhibited production of dark neurons in the hippocampal CA1 and CA3 areas as well as somatosensory cortex in rats after induction of seizures by intraperitoneal pentylenetetrazol (PTZ) injection. * indicates p<0.001.

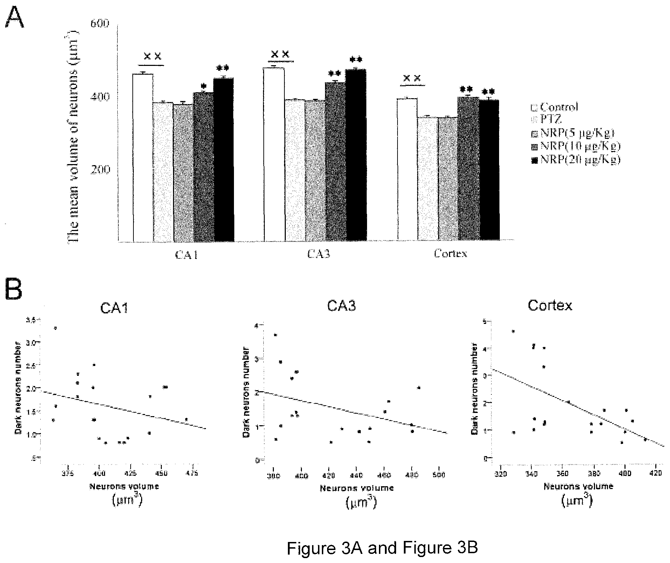

Figure 3A. Shows a plot of the effects of different concentrations of NRP2945 administration on volume-weighted mean volume of normal neurons in hippocampal CA1 and CA3 areas and the somatosensory cortex after PTZ-induced seizures in rats. The mean volume-weighted volume of normal neurons after treatment with NRP2945 at concentration of 10 and 20 µg/kg was higher in different brain regions compared to control rats. A
Figure 3B. Shows graphically the effects of different concentrations of NRP2945 administration on volume-weighted mean volume of normal neurons in hippocampal CA1 and CA3 areas and the somatosensory cortex after PTZ-induced seizures in rats. Correlation between the volume-weighted mean volume of normal neurons and the number of dark neurons revealed that an increase in the volume-weighted mean volume of normal neurons was accompanied by a decrease in the number of dark neurons in hippocampal CA1 and CA3 areas as well as in the somatosensory cortex after application of NRP2945. * indicates p< 0.001.

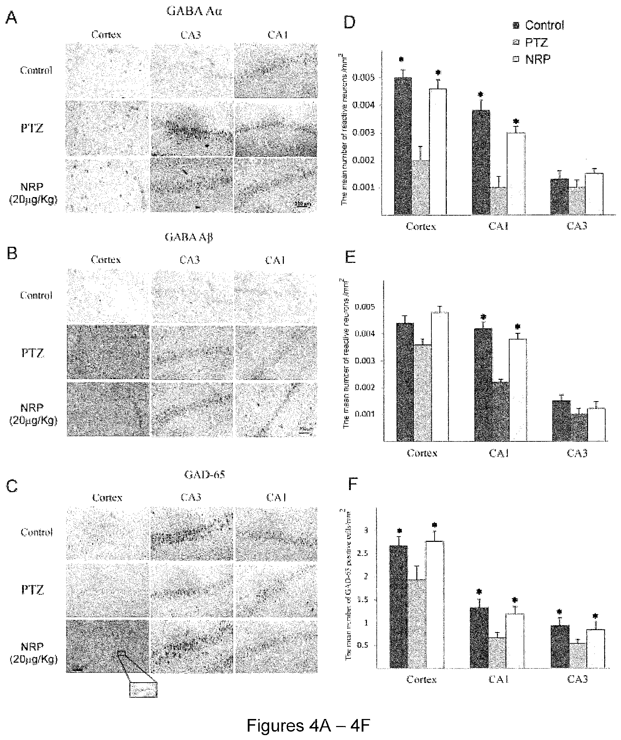

Figures 4A-4C show photomicrographs of immunohistochemistry of GABAA α and β receptors as well as GAD-65 expression in the neocortex and hippocampal CA1 and CA3 areas. GABAA α and β receptors as well as GAD-65 expression in the hippocampal CA1 and CA3 regions and in the somatosensory cortex after application of NRP2945 (20 µg/kg) thirty minutes before PTZ injection.
Figures 4D-4F show bar graphs that illustrate the quantitative results (mean ± S.E.M) of GABAA α and β receptors as well as GAD-65 expression in the neocortex and hippocampal CA1 and CA3 areas. Data indicate treatment with NRP2945 before PTZ injection resulted in a higher expression of GABAA α subunit in the neocortex and CA1 hippocampal region. GABAA β subunit expression was higher only in CA1 area. GAD-65 expression was significantly higher in both the neocortex and hippocampal CA1 and CA3 regions after treatment with NRP2945 compared to the control rats.* indicate p< 0.001.
Further aspects of the disclosure will become apparent with reference to the accompanying Figures and Example described below:

### Example 1: Materials and Methods

Adult male wistar rats (200-250g) were housed under controlled conditions (ambient temperature = 22°C, humidity = 40%; 12-h light/dark cycle) with food and water ad libitum. Animals were age- and weight-matched and randomly received by intra peritoneal injection NRP2945 in saline (5, 10 or 20 µg/kg) or saline alone (control) thirty minutes before the i.p. injection of pentylenetetrazol (PTZ; 50 mg/kg dissolved in 0.9% saline). All experiments were carried out according to the protocol approved by the Animal Ethics Committee of Shefa Neuroscience Center, Tehran, Iran.

### Behavioural tests

After each PTZ-injection, the convulsive behaviour of each rat was assessed for 30 min, and resultant seizures were scored as follows: 0 = normal behaviour; 1 = immobility; 2 = rigid posture; 3 = repetitive scratching, circling, or head bobbing; 4 = forelimb clonus, rearing, and falling; 5 = repeated occurrence of level four behaviour, 6 = severe tonic-clonic behaviour or status epilepticus (Karimzadeh et al., 2013). Latency of seizure onset was defined as the average length of time in seconds between PTZ administration an initially detectable and seizure. A generalized seizure was characterised by symmetric forelimb and hind limb tonus, followed by hind limb clonus and subsequent jumping activity. Because some animals occasionally had more than one seizure, the seizure duration was assessed as the sum of durations of these multiple seizures.

### Surgical Procedure and Electrocorticogram (ECoG) recording

Animals were anaesthetized with chloral hydrate 3.5% (dissolved in normal 0.9% saline; Sigma-Aldrich) and the head of each rat was placed in a stereotaxical instrument (Stoelting Instruments, USA). Epidural recording electrodes were stereotaxically implanted above the left and right somatosensory cortices (3.0 mm posteriorly to Bregma and 3.0 mm laterally from the midline) under continuous chloral hydrate anesthesia. The reference electrode is located at the nasal bone. ECoG was recorded via monopolar silver electrodes connected to an amplifier (EXT-02 F, NPI, Germany; with band-pass filters at 0.5-30 kHz, sampling rate 10 kHz) and stored by a digital oscilloscope. ECoG recordings were performed for 30 minutes after receiving saline or NRP2945 (5, 10, 20 µg/kg) and 30 minutes after the injection of PTZ. Latency, duration, amplitude, and frequency of epileptic spikes activity were calculated using AxoScope 10 software (Axon Instruments, CA, USA).

### Stereological methods and physical dissector

The volume-weighted mean volume of normal neurons was calculated directly by point-sampled intercept on 10 uniform systematically and randomly sampled microscopic fields of the pyramidal layers of hippocampal areas CA1 and CA3 as well as the somatosensory cortex. A lattice of test points on lines was superimposed randomly onto the traced nuclear profiles in each particular field. Nuclei of neurons were marked and two isotropic lines from randomly selected directions were centred on this neuron and superimposed. The intersection of each line with the outer surface of the respective neuron's soma was marked. These lines produced point-sampled intercepts, the lengths of which were measured and cubed. The mean was multiplied by π/3, and finally all intercepts were averaged to give an estimate of the volume-weighted mean neuronal volume. For quantitative analysis of dark neurons, the physical dissector method was used. Ten pairs of sections, with 8-mm distance, were collected from each brain. The first section of each pair was designated as the reference and the second one was used for comparison. On each pair of sections, at least 10 microscopic fields were selected by uniform systematic-random sampling in every area of interest. Using the unbiased frame and physical dissector counting rule, the counting of dark neurons in each field was carried out (Jafarian et al., 2010).

### Histological studies

Animals were deeply anaesthetised with chloral hydrate (3.5%; Sigma-Aldrich) and perfused transcardially with 200 ml of saline followed by 200 ml of a solution containing 1% paraformaldehyde (PFA). After perfusion, all rats were decapitated and the brains were removed. The brains were kept in 1% PFA for at least 10 days and were then processed for histological studies as follows. The brain was cut into transverse sections of 8 µm thickness. Sections were stained with toluidine blue. The sections were selected by uniform systematic random sampling in every area of interest. Different areas (hippocampal CA1 and CA3 areas and somatosensory neocortex) were studied under a light microscope (BX51, Olympus, Japan) equipped with a digital camera. Digital photographs were taken using a 40× oil immersion objective lens (Olympus, Japan). The magnification was calculated using an objective micrometer.

### Immunohistochemistry method

Animals were deeply anaesthetized with chloral hydrate and perfused transcardially with 200 ml of saline followed by 600 ml of 4% PFA solution. The brains were extracted and kept in 4% PFA for at least 4 days at 4 ºC and were then processed for immunohistochemistry studies. The serial (8 µm) coronal sections were prepared. Three paraffin embedded sections in 1.8 to 3.3 mm posterior to the bregma from each animal were cleared and rehydrated through a series of xylol and alcohol and washed with phosphate buffered saline (PBS) three times and incubated in blockage solution (3% H2O2 /methanol for 5 min). After washing in PBS, slides were boiled in sodium citrate buffer at 95 ºC for 10 min. After cooling to room temperature for 20 min, slides were washed with PBS three times and incubated overnight at 4 ºC with commercial rabbit polyclonal anti-rat antibody (Abcam, Cambridge, United Kingdom) against GABAA α, GABAA β or GAD-65 overnight. Antibody concentration for GABAA α was 1:100, for GABAA β was 1: 50 and for Glutamic acid decarboxylase-65 (GAD-65) was 1:200 in a solution containing 1-5% NGS in 0.3% Triton X-100 and 0.1 M PBS at pH 7.4. The sections were then rinsed three times in PBS (10 min each), and incubated with goat anti-rabbit horseradish peroxidase-conjugated secondary antibody (Abcam, Cambridge, United Kingdom) diluted at 1:400 in PBS with 0.3% Triton X-100 and 5% NGS at 22 ºC for 1 h. After several washings with PBS, the slides were incubated and developed in 3-3'-diaminobenzidine (DAB, Roche; 0.5 µl DAB and 1.5 µl peroxide buffer) for 5-10 min. Sections were counterstained with haematoxylin. Control for the specificity of immunostaining was performed by omission of the primary antibody. Images for analysis were acquired with a digital camera attached to the microscope.

Data were expressed as mean ± SEM. Statistical analysis was carried out by using one-way analysis of variance (ANOVA) followed by Tukey's post-hoc test and LSD test. The criterion for statistical significance was P < 0.05. Correlation between the number of dark neurons and the volume of neurons was analysed by Pearson's test.

### Results

### (i) The effect of NRP2945 on PTZ-induced seizures

In the present study, PTZ was used to induce epileptic seizures in rats. All animals of the control group developed typical seizure behaviour about 2-3 min following PTZ injection. PTZ-induced seizures or convulsions were completely prevented by administration of NRP2945 at concentration of 20µg/kg. At lower concentrations (5 and 10µg/Kg), NRP2945 reduced the total duration of seizure attacks after PTZ injection compared to control animals (p < 0.05, n = 12; Fig. 1 A, and see Table 1) but was not affecting the latency and severity of seizure attacks (Fig. 1 A).

| | **Control** | **5 µg/kg** | **10 µg/kg** | **20 µg/kg** |
|---|---|---|---|---|
| **Behavioural test** | | | | |
| **Latency** (sec) | 144.2 ± 11.8 | 230.2 ± 10.6 | 150.2 ± 7.4 | - |
| **Duration** (sec) | 60.5 ± 0.5 | 35.83 ± 5.9^{∗} | 37.67± 7.3^{∗} | - |
| **Severity** | 4 ± 0 | 3.67 ± 0.21 | 3.67 ± 0.21 | - |

| **ECoG** | | | | |
|---|---|---|---|---|
| **Latency** (sec) | 27 ± 7 | 326 ± 24^{∗∗} | 417 ± 37^{∗∗} | - |
| **Duration** (sec) | 180 ± 7.2 | 60 ± 1.2^{∗∗} | 60 ± 2.1^{∗∗} | - |
| **Amplitude** (mV) | 1.4 ± 0.1 | 1.1 ± 0.04^{∗∗} | 0.84 ± 0.1^{∗∗} | - |
| **Frequency** (per min) | 15 ± 1.2 | 4.7 ± 0.3^{∗∗} | 6.67 ± 0.9^{∗∗} | - |

| **Dark neurons** | | | | |
|---|---|---|---|---|
| **CA1** | 2.56 ± 0.2 | 1.53 ± 0.1^{∗∗} | 0.88 ± 0.04^{∗} | 1.37 ± 0.1^{∗∗} |
| **CA3** | 2.70 ± 0.1 | 1.31 ± 0.2^{∗∗} | 0.80 ± 0.1^{∗∗} | 1.26 ± 0.1^{∗∗} |
| **Cortex** | 3.63 ± 0.3 | 1.42 ± 0.1^{∗∗} | 0.78 ± 0.1^{∗∗} | 1.30 ± 0.1^{∗∗} |

| **Mean volume of normal neurons** | | | | |
|---|---|---|---|---|
| **CA1** | 384.9 ± 5.6 | 380.9 ± 7.1 | 413.1 ± 4.4^{∗∗} | 451.9 ± 5.5^{∗∗} |
| **CA3** | 390.9 ± 3.1 | 387.9 ± 3.2 | 438.6 ± 5.6^{∗∗} | 474.1 ± 5.1^{∗∗} |
| **Cortex** | 341.9 ± 3.6 | 340.8 ± 3.1 | 395.7 ± 6.1^{∗∗} | 387.9 ± 7.5^{∗∗} |

Table 1. Tabulates the effect of NRP2945 at concentrations of 5, 10 and 20 µg/kg on behaviour (PTZ-induced seizure attacks), epileptiform burst discharges recorded by electrocorticography (ECoG), the mean number of dark neurons and the mean volume of normal neurons after injection of PTZ. The control group was only treated with PTZ. NRP2945 at different concentrations was administered in saline i.p. thirty minutes before PTZ injection.* and ** indicate p<0.05 and p<0.001, respectively.

### (ii) The effect of NRP2945 on PTZ-induced epileptiform ECoG activity

ECoG recording in anesthetized rats was performed to validate the behavioural observations. Recordings in control rats consisted of a series of surface positive flat topped deflections. These waves had durations of 20 to 200 ms and a frequency of 0.3 to 4 Hz. Following injection of PTZ (50 mg/kg), the first epileptiform spike occurred within 20 to 185 (45 ± 11) seconds of the injection. The spikes steadily increased in size and reached amplitudes of 1.4 ± 0.1 mV, durations of 180 ± 7.2 sec and an occurrence of 15 ± 1.2/min within 5-10 minutes. The epileptiform burst discharges then remains constant for another 30 minutes (control group). Application (i.p.) of NRP2945 in saline at a concentration of 20µg/kg thirty minutes before PTZ injection completely prevented development of epileptiform field potentials (Fig. 1 B). NRP2945 at concentrations of 5 and 10 µg/kg increased the latency time to onset of first spike waves and decreased the amplitude, duration and repetition rate of the epileptiform field potentials induced by PTZ (P≤0.001; Fig. 1 B and C).

### (iii) The effect of NRP2945 on production of dark neurons

The appearance of eosinophilic neurons with condensed darkly stained nuclei and bright eosinophilic cytoplasm was used to assess neuronal degeneration following PTZ treatment in the following (Garman, 2011). Dark neurons were identified by neuronal shrinkage, cytoplasmic eosinophillia, nuclear pyknosis, and surrounding spongiosis (Jafarian et al., 2010). Density of dark neurons in the hippocampal CA1 and CA3 areas and somatosensory cortex was significantly decreased after administration of NRP2945 in comparison to saline administration. The mean number of dark neurons in the hippocampal CA1 area decreased from 2.56 ± 0.23 in control rats (received only PTZ) to 1.53 ± 0.13 , 0.88 ± 0.04 and 1.37 ± 0.09 after application of 5, 10 and 20 µg/kg, respectively (P≤0.001; Fig. 2 A and B, Table 1). The mean number of dark cells in the hippocampal CA3 area also significantly reduced from 2.7 ± 0.14 in control rats to 1.31 ± 0.17, 0.8 ± 0.07 and 1.26 ± 0.11 after application of 5, 10 and 20 µg/kg, respectively (P≤0.001; Fig. 2, Table). NRP2945 also reduced production of dark neurons by PTZ injection in the neocortex. The mean number of dark cells in the somatosensory cortex in control group was 3.63 ± 0.25. Administration of NRP2945 at concentrations of 20 µg/kg before injection of PTZ reduced the number of these neurons to 1.42 ± 0.12, 0.78 ± 0.09, 1.3 ± 0.08, respectively (P≤0.001; Fig. 2 A and B, Table 1).

### (iv) The effect of NRP2945 on the mean volume of normal neurons

There was a significant difference in the volume-weighted mean volume of normal neurons observed in the hippocampus and in the somatosensory cortex after application of different concentrations of NRP2945 in comparison with control rats (Fig. 3 A, Table 1). NRP2945 at 5 µg/kg did not affect the mean volume of normal neurons after PTZ-induced seizures. The hippocampal CA1 and CA3 areas and the somatosensory cortex had a larger mean volume-weighted volume of normal neurons after application of NRP2945 at 10 and 20µg/kg compared to the control group (P≤0.001; Fig. 3 A, Table 1).

Correlation analysis between the volume-weighted mean volume of normal neurons and the number of dark neurons revealed that an increase in the volume-weighted mean volume of normal neurons was accompanied by a decrease in the number of dark neurons in the hippocampal CA1 (r=0.084, P≤ 0.001) and CA3 (r=0.147, P≤ 0.0001) areas as well as in the somatosensory cortex (r=0.302, P≤0.001) after application of NRP2945 (Fig. 3 B).

### (v) The effect of NRP2945 on GABA A receptor α and β subunits and GAD-65

To evaluate a possible mechanism of the anticonvulsant effect of NRP2945, the expression of GABAA receptor α and β subunits and GAD-65 after PTZ injection was assessed by immunohistochemical staining. A significant enhancement in the expression of GABAA receptor α subunit in the somatosensory cortex and hippocampal CA1 area following treatment with NRP2945 (20 µg/kg) was observed when compared to the control rats (P < 0.001; Fig. 4A). Application of NRP2945 at 20µg/kg only enhanced the expression of GABAA receptor β subunit in the CA1 hippocampal region compared to control tissues (P < 0.001; Fig. 4B). We also examined the effects of NRP2945 on GAD-65 immunoreactivity in the hippocampus and neocortex. The immunohistochemical labelling for GAD-65 in the somatosensory cortex as well as in the CA1 and CA3 hippocampal areas revealed a significant increase in the expression of GAD-65 immunoreactive neurons in these brain regions after application of NRP2945 (20µg/kg) compared to the control rats (P < 0.001; Fig. 4C).

The results strongly suggest that NRP2945 provides anticonvulsant and neuroprotective effects on the PTZ-induced seizure activities and epileptiform field potentials and that this peptide may provide a desirable option for preventing epilepsy or seizures. In addition, the results also strongly suggest that NRP2945 prevents damage to cell structure produced by seizure activities after PTZ injection. NRP2945 reduced the number of dark neurons and increased the mean volume of normal neurons in different brain regions. A strong correlation between prevention of cell injury and enhancement of the mean volume of normal neurons by NRP2945 was observed. Furthermore, NRP2945 enhanced expression of GABA A receptor α and β subunits and GAD-65 in the hippocampus and somatosensory cortex in rats receiving PTZ injection.

In particular, it is anticipated that functionally similar peptide sequences may be obtained by substitution of one or more amino acids of the biosequence with a functionally similar amino acid. It is suggested that the functionality of similar peptide sequences may be confirmed without undue additional experimentation by use of the method disclosed in this specification.

### REFERENCES

Gorba T, Bradoo P, Antonic A, Marvin K, Liu DX, Lobie PE, Reymann KG, Gluckman PD, Sieg F. Neural regeneration protein is a novel chemoattractive and neuronal survival-promoting factor. Exp Cell Res. 2006 Oct 1;312(16):3060-74.
Sieg F, Wahle P, Pape HC. Cellular reactivity to mechanical axonal injury in an organotypic in vitro model of neurotrauma. Journal of Neurotrauma. 1999, 16: 1197-1
Landgraf P, Sieg F, Meyer G, Wahle P, Pape HC, Kreutz MR. A maternal blood-borne factor promotes survival of developing thalamus. FASEB J. 2005, 19: 225-227.
Sadakata T, Mizoguchi A, Sato Y, Katoh-Semba R, Fukuda M, Mikoshiba K, Furuichi T. The secretory granule-associated protein CAPS2 regulates neurotrophin release and cell survival. J Neurosci. 2004 Jan 7;24(1):43-52.
Speidel D, Varoqueaux F, Enk C, Nojiri M, Grishanin RN, Martin TF, Hofmann K, Brose N, Reim K. A family of Ca2+-dependent activator proteins for secretion: comparative analysis of structure, expression, localization, and function. J Biol Chem. 2003 Dec 26;278(52):52802-9.
Sieg F, Antonic A. Alternative Strategies in Neuroregeneration and Neurogenesis. Research signpost edition 2007 (chapter, pp 27-58). Editors: Valeria Sogos and Andrea Diana.
Singh, AT, Keelan JA, Sieg F. Regulation of trophoblast migration and survival by a novel neural regeneration peptide (NRP). Reprod. Biomed. Online. 2010 Aug;21(2):237-44.
Aronica E, Becker AJ, Spreafico, R. Malformations of cortical development. Brain Pathol. 2012 May;22(3):380-401.
Choi J, Koh S. Role of brain inflammation in epileptogenesis. Yonsei Med J. 2008 Feb 29;49(1):1-18.
Vezzani A, Ruegg S. The pivotal role of immunity and inflammatory processes in epilepsy is increasingly recognized: introduction. Epilepsia. 2011 May;52 Suppl 3:1-4.
Karimzadeh F, Soleimani M, Mehdizadeh M, Jafarian M, Mohamadpour M, Kazemi H, Joghataei MT, Gorji A. Diminution of the NMDA receptor NR2B subunit in cortical and subcortical areas of WAG/Rij rats. Synapse. 2013 Dec;67(12):839-46.
Jafarian M, Rahimi S, Behnam F, Hosseini M, Haghir H, Sadeghzadeh B, Gorji A. The effect of repetitive spreading depression on neuronal damage in juvenile rat brain. Neuroscience. 2010 Aug 11;169(1):388-94.
Ferrendelli JA, Holland, KD, McKeon AC, Covey DF. Comparison of the anticonvulsant activities of ethosuximide, valproate, and a new anticonvulsant, thiobutyrolactone. Epilepsia. 1989 Sep-Oct;30(5):617-22.
Meldrum B. Do preclinical seizure models preselect certain adverse effects of antiepileptic drugs. Epilepsy Res. 2002 Jun;50(1-2):33-40.
Kupferberg H. Animal models used in the screening of antiepileptic drugs. Epilepsia 2001;42 Suppl 4:7-12.
Leweke FM, Louvel J, Rausche G, Heinemann U. Effects of pentetrazol on neuronal activity and on extracellular calcium concentration in rat hippocampal slices. Epilepsy Res. 1990 Aug;6(3):187-98.
Walsh TJ, Stackman RW, Emerich DF, Taylor LA. Intraseptal injection of GABA and benzodiazepine receptor ligands alters high-affinity choline transport in the hippocampus. Brain Res Bull. 1993;31(3-4):267-71.
Follesa P, Tarantino A, Floris S, Mallei A, Porta S, Tuliqi G, Cagetti E, Caddeo M, Mura A, Serra M, Biggio G. Changes in the gene expression of GABAA receptor subunit mRNAs in the septum of rats subjected to pentylenetetrazol-induced kindling. Brain Res Mol Brain Res. 1999 Jun 18;70(1):1-8.
Psarropoulou C, Matsokis N, Anquelatou F, Kostopoulos G. Pentylenetetrazol-induced seizures decrease gamma-aminobutyric acid-mediated recurrent inhibition and enhance adenosine-mediated depression. Epilepsia 1994 Jan-Feb;35(1):12-9.
Marley RJ, Heninger C, Hernandez TD, Gallager TW. Chronic administration of beta-carboline-3-carboxylic acid methylamide by continuous intraventricular infusion increases GABAergic function. Neuropharmacology 1991 Mar;30(3):245-51.
Rocha L, Ackermann RF, Engel JJr. Chronic and single administration of pentylenetetrazol modifies benzodiazepine receptor-binding: an autoradiographic study. Epilepsy Res. 1996 Jun;24(2):65-72.
El Idrissi A, Messing J, Scalia J, Trenkner E. Prevention of epileptic seizures by taurine. Adv Exp Med Biol. 2003;526:515-25.
Becker A, Grecksch G, Matthies H. The influence of diazepam on learning processes impaired by pentylenetetrazol kindling. Naunyn Schmiedebergs Arch Pharmacol. 1994 May;349(5):492-6.
Gasior M, Ungard JT, Beekman M, Carter RB, Witkin JM. Acute and chronic effects of the synthetic neuroactive steroid, ganaxolone, against the convulsive and lethal effects of pentylenetetrazol in seizure-kindled mice: comparison with diazepam and valproate. Neuropharmacology 2000 Apr 27;39(7):1184-96.
Tirassa P, Costa N, Aloe L. CCK-8 prevents the development of kindling and regulates the GABA and NPY expression in the hippocampus of pentylenetetrazole (PTZ)-treated adult rats. Neuropharmacology. 2005 Apr;48(5):732-42.
He X, Wang W, Ruan X, Li W, Zhang L. Effects of antisense glutamic acid decarboxylase oligodeoxynucleotide on epileptic rats induced by pentylenetetrazol. Chin Med J (Engl). 2002 Mar;115(3):425-9.
Holmes GL, Sarkisian M, Ben-Ari Y, Chevassus-Au-Louis N. Mossy fiber sprouting after recurrent seizures during early development in rats. J Comp Neurol. 1999 Feb 22;404(4):537-53.
Huang LT, Yang SN, Liou CW, Hung PL, Lai MC, Wang CL, Wang TJ. Pentylenetetrazol-induced recurrent seizures in rat pups: time course on spatial learning and long-term effects. Epilepsia 2002 Jun;43(6):567-73.
Mortazavi F, Ericson M, Story D, Hulce VD, Dunbar GL. Spatial learning deficits and emotional impairments in pentylenetetrazole-kindled rats. Epilepsy Behav. 2005 Dec;7(4):629-38. Epub 2005 Oct 24.
Fang F, Lei H. Increased hippocampal T2 in a rat model of pentylenetetrazol-induced kindling correlates with seizure scores. J Neurol Sci. 2010; 292(1-2):16-23.
Franke H, Kittner H. Morphological alterations of neurons and astrocytes and changes in emotional behavior in pentylenetetrazol-kindled rats. Pharmacol Biochem Behav. 2001 Oct-Nov;70(2-3):291-303.
Rauca C, Zerbe R, Jantze H. Formation of free hydroxyl radicals after pentylenetetrazol-induced seizure and kindling. Brain Res., 847, 347-351 (1999).
Patsoukis N, Zervoudakis G, Georgiou CD, Angelatou F, Matsokis NA, Panagopoulos NT. Effect of pentylenetetrazol-induced epileptic seizure on thiol redox state in the mouse cerebral cortex. Epilepsy Res 2004 Nov;62(1):65-74.
Tuunanen J, Halonen T, Pitkaenen A. Status epilepticus causes selective regional damage and loss of GABAergic neurons in the rat amygdaloid complex. Eur J Neurosci. 1996 Dec;8(12):2711-25.
Karimzadeh, F, Hosseini M, Mangeng D, Alavi H, Hassanzadeh GR, Bayat M, Jafarian M, Kazemi H, Gorji A. Anticonvulsant and neuroprotective effects of Pimpinella anisum in rat brain. BMC Complement Altern Med. 2012 Jun 18;12:76.
Ito H, Watanabe Y, Isshiki A, Uchino H. Neuroprotective properties of propofol and midazolam, but not pentobarbital, on neuronal damage induced by forebrain ischemia, based on the GABAA receptors. Acta Anaesthesiol Scand. 1999 Feb;43(2):153-62.
Garman RH. Histology of the central nervous system. Toxicol Pathol. 2011 Jan;39(1):22-35.

## Claims

1. A peptide comprising the sequence:
GlyArgArgAlaAlaProGlyArgAibGlyGly (SEQ ID NO:1)
for use in a method of preventing
(i) epilepsy; or
(ii) a seizure;
which method comprises administering an effective amount of the peptide to a subject in need thereof.

2. . The peptide for use as claimed in claim 1, wherein the method is a method of (i) increasing latency, (ii) decreasing duration, (iii) decreasing severity, (iv) decreasing amplitude, and/or (v) decreasing frequency, of an epileptiform discharge in a subject.

3. The peptide for use as claimed in claim 1 or claim 2, wherein the peptide consists of the 11 amino acid residue sequence:
GlyArgArgAlaAlaProGlyArgAibGlyGly (SEQ ID NO:1)
or functionally acceptable derivatives thereof.

4. The peptide for use as claimed in claim 3, wherein the C-terminus of the peptide is amidated to give:
GlyArgArgAlaAlaProGlyArgAibGlyGly-NH₂(SEQ ID NO:2).

5. The peptide for use as claimed in any one of claims 1 to 4, wherein the administration step to the subject is by way of injection, including but not limited to intraperitoneal, intravenous, intramuscular, intraarterial, subcutaneous, intradermal, intracerebroventricular, intranasal and intraspinal injection.

6. The peptide for use as claimed in any one of claims 1 to 4, wherein the administration step to the subject is by way of oral administration.

7. The peptide for use as claimed in any one of claims 1 to 6, wherein the effective amount of the peptide is at least 5µg/kg administered to the subject.

8. The peptide for use as claimed in any one of claims 1 to 7, wherein the effective amount of the peptide is about 10-50µg/kg administered to the subject.

9. The peptide for use as claimed in any one of claims 1 to 8, wherein the effective amount of the peptide comprising the sequence:
GlyArgArgAlaAlaProGlyArgAibGlyGly (SEQ ID NO:1) or
GlyArgArgAlaAlaProGlyArgAibGlyGly-NH₂(SEQ ID NO:2)
is administered to a subject in need thereof on an at least once a day basis.

10. The peptide for use as claimed in claim 9, wherein the effective amount of the peptide comprising the sequence:
GlyArgArgAlaAlaProGlyArgAibGlyGly (SEQ ID NO:1)
is administered to a subject in need thereof on an at least once a day basis.

11. The peptide for use as claimed in claim 9 or claim 10, wherein the effective amount of the peptide is administered on an at least twice a day basis.

## Patentansprüche

1. Peptid, das die folgende Sequenz umfasst:
GlyArgArgAlaAlaProGlyArgAibGlyGly (SEQ ID NO: 1),
zur Verwendung in einem Verfahren zur Vorbeugung von
(i) Epilepsie; oder
(ii) einem Anfall;
wobei das Verfahren das Verabreichen einer wirksamen Menge des Peptids an ein bedürftiges Individuum umfasst.

2. Peptid zur Verwendung nach Anspruch 1, wobei das Verfahren ein Verfahren zur (i) Erhöhung der Latenz, (ii) Verringerung der Dauer, (iii) Verringerung der Schwere, (iv) Verringerung der Amplitude und/oder (v) Verringerung der Häufigkeit einer epileptiformen Entladung in einem Individuum ist.

3. Peptid zur Verwendung nach Anspruch 1 oder Anspruch 2, wobei das Peptid aus der 11 Aminosäurereste umfassenden Sequenz
GlyArgArgAlaAlaProGlyArgAibGlyGly (SEQ ID NO: 1)
oder funktional annehmbaren Derivaten davon besteht.

4. Peptid zur Verwendung nach Anspruch 3, wobei der C-Terminus des Peptids amidiert ist, um Folgendes zu erhalten:
GlyArgArgAlaAlaProGlyArgAibGlyGly-NH₂ (SEQ ID NO: 2).

5. Peptid zur Verwendung nach einem der Ansprüche 1 bis 4, wobei der Schritt der Verabreichung an das Individuum mittels Injektion erfolgt, einschließlich, ohne Einschränkung, intraperitonealer, intravenöser, intramuskulärer, intraarterieller, subkutaner, intradermaler, intracerebroventrikulärer, intranasaler und intraspinaler Injektion.

6. Peptid zur Verwendung nach einem der Ansprüche 1 bis 4, wobei der Schritt der Verabreichung an das Individuum mittels oraler Verabreichung erfolgt.

7. Peptid zur Verwendung nach einem der Ansprüche 1 bis 6, wobei die wirksame Menge des Peptids zumindest 5 µg/kg beträgt, die dem Individuum verabreicht wird.

8. Peptid zur Verwendung nach einem der Ansprüche 1 bis 7, wobei die wirksame Menge des Peptids etwa 10-50 µg/kg beträgt, die dem Individuum verabreicht wird.

9. Peptid zur Verwendung nach einem der Ansprüche 1 bis 8, wobei die wirksame Menge des Peptids, das die folgende Sequenz umfasst:
GlyArgArgAlaAlaProGlyArgAibGlyGly (SEQ ID NO: 1) oder
GlyArgArgAlaAlaProGlyArgAibGlyGly-NH₂ (SEQ ID NO: 2),
einem bedürftigen Individuum zumindest einmal täglich verabreicht wird.

10. Peptid zur Verwendung nach Anspruch 9, wobei die wirksame Menge des Peptids, das die folgende Sequenz umfasst:
GlyArgArgAlaAlaProGlyArgAibGlyGly (SEQ ID NO: 1),
einem bedürftigen Individuum zumindest einmal täglich verabreicht wird.

11. Peptid zur Verwendung nach Anspruch 9 oder Anspruch 10, wobei die wirksame Menge des Peptids zumindest zweimal täglich verabreicht wird.

## Revendications

1. Peptide comprenant la séquence :
GlyArgArgAlaAlaProGlyArgAibGlyGly (SEQ ID NO:1)
à utiliser dans un procédé de prévention
(i) de l'épilepsie ; ou
(ii) d'une crise d'épilepsie ;
le procédé comprenant l'administration d'une quantité efficace du peptide à un sujet qui en a besoin.

2. Peptide à utiliser selon la revendication 1, dans lequel le procédé est un procédé (i) d'augmentation de la latence, (ii) de diminution de la durée, (iii) de diminution de la gravité, (iv) de diminution de l'amplitude, et/ou (v) de diminution de la fréquence, d'une décharge épileptiforme chez un sujet.

3. Peptide à utiliser selon la revendication 1 ou la revendication 2, dans lequel le peptide est constitué de la séquence de résidus d'acides aminés 11 :
GlyArgArgAlaAlaProGlyArgAibGlyGly (SEQ ID NO:1)
ou de dérivés fonctionnellement acceptables de celui-ci.

4. Peptide à utiliser selon la revendication 3, dans lequel l'extrémité C-terminale du peptide est amidée pour donner :
GlyArgArgAlaAlaProGlyArgAibGlyGly-NH₂(SEQ ID NO:2).

5. Peptide à utiliser selon l'une quelconque des revendications 1 à 4, dans lequel l'étape d'administration au sujet est par injection, y compris, mais sans s'y limiter, une injection intrapéritonéale, intraveineuse, intramusculaire, intra-artérielle, sous-cutanée, intradermique, intracérébroventriculaire, intranasale et intraspinale.

6. Peptide à utiliser selon l'une quelconque des revendications 1 à 4, dans lequel l'étape d'administration au sujet se fait par voie orale.

7. Peptide à utiliser selon l'une quelconque des revendications 1 à 6, dans lequel la quantité efficace du peptide administrée au sujet est d'au moins 5 µg/kg.

8. Peptide à utiliser selon l'une quelconque des revendications 1 à 7, dans lequel la quantité efficace du peptide administrée au sujet est d'environ 10 à 50 µg/kg.

9. Peptide à utiliser selon l'une quelconque des revendications 1 à 8, dans lequel la quantité efficace du peptide comprenant la séquence :
GlyArgArgAlaAlaProGlyArgAibGlyGly (SEQ ID NO:1) ou
GlyArgArgAlaAlaProGlyArgAibGlyGly-NH₂(SEQ ID NO:2)
est administré à un sujet qui en a besoin sur une base d'au moins une fois par jour.

10. Peptide à utiliser selon la revendication 9, dans lequel la quantité efficace du peptide comprenant la séquence :
GlyArgArgAlaAlaProGlyArgAibGlyGly (SEQ ID NO:1)
est administrée à un sujet qui en a besoin sur une base d'au moins une fois par jour.

11. Peptide à utiliser selon la revendication 9 ou la revendication 10, dans lequel la quantité efficace du peptide est administrée sur une base d'au moins deux fois par jour.
